# EUROPEAN PATENT APPLICATION

(11) **EP 4 595 936 A1**
(43) Date of publication of application: **06.08.2025**
(21) Application number: 24218271.5
(22) Date of filing: 09.12.2024
(51) Int. Cl.: A61F 13/15, A61F 13/495

(54) **AN APPARATUS AND METHOD FOR PRODUCING ELASTIC SEGMENTS**

(30) Priority: 05.02.2024 IT 202400002343
(71) Applicant: Fameccanica.Data S.p.A., 66020 San Giovanni Teatino (CH) (IT)
(72) Inventor: SABLONE, Gabriele, 66020 San Giovanni Teatino (Chieti) (IT)
(74) Representative: Buzzi, Notaro & Antonielli d'Oulx S.p.A.

(57) **Abstract**

An apparatus for producing strip segments, comprising a film feeding unit (26) and a thread feeding unit (30), selectively operated to feed either a continuous elastic film (28) or a plurality of elastic threads (32) between a first and a second non-woven fabric web (16, 18), thereby forming, selectively, an array of elastic film segments (40) or an array of elastic thread segments (42).

## Description

### Field of the Invention

The present invention relates to an apparatus and a method for producing elastic segments.

The invention was developed particularly with a view to its application in machines for manufacturing absorbent sanitary articles.

Throughout this description, reference will be made to this field of application without, however, losing general applicability.

The present invention also relates to a machine for manufacturing absorbent sanitary articles comprising an apparatus for producing elastic segments.

### Prior Art

In machines for manufacturing absorbent sanitary articles, it is often necessary to produce elastic segments.

Elastic segments used in the production of absorbent sanitary articles usually consist of two non-elastic layers of non-woven fabric bonded together with an elastic structure enclosed between the two layers of non-woven fabric. These elastic segments can be either elastic film segments or elastic thread segments.

In elastic film segments, the elastic structure is made up of an elastic film sandwiched between two non-elastic layers of non-woven fabric, for example, through a pattern of bonding points.

In elastic thread segments, the elastic structure consists of multiple elastic threads arranged parallel to each other and secured between two non-elastic layers of non-woven fabric via a pattern of anchoring bonds, as described, for example, in EP-A-3092997 by the same applicant, or through a pattern of glue dots.

Elastic segments of this kind can be used to create containment elements applied to the waistband of absorbent sanitary articles to form pockets for containing feces, as described, for example, in EP-A-4159171 by the same applicant.

Elastic film segments are produced by providing a continuous elastic film that is stretched elastically in a direction transverse to the longitudinal axis of the continuous elastic film. The transversely stretched elastic film is sandwiched between two non-elastic webs of non-woven fabric. The non-woven fabric webs and the transversely stretched elastic film are bonded together through a pattern of bonding points to form a composite elastic web, which is then transversely cut to create individual elastic film segments.

Elastic thread segments are produced by providing a plurality of elastic threads stretched elastically in the longitudinal direction. The longitudinally stretched elastic threads are arranged parallel to one another and sandwiched between two non-elastic webs of non-woven fabric. The non-woven fabric webs and the longitudinally stretched elastic threads are bonded together through a pattern of bonding points or glue dots to form a composite elastic web, which is then transversely cut to create individual elastic thread segments.

Machines for manufacturing absorbent sanitary articles according to the prior art that use elastic segments are equipped either with an apparatus for producing elastic film segments or with an apparatus for producing elastic thread segments.

In the prior art, it is not possible to produce absorbent sanitary articles with elastic film segments and absorbent sanitary articles with elastic thread segments using the same machine. Therefore, if a manufacturer wishes to produce both types of articles, they must use two separate machines, resulting in significantly higher costs.

### Object and Summary of the Invention

The object of the present invention is to provide an apparatus and a method for producing strip segments that overcome the problems of the prior art.

According to the present invention, this object is achieved by an apparatus having the features forming the subject of claim 1 and by a method having the features forming the subject of claim 6.

According to another aspect, the present invention also relates to a machine for manufacturing absorbent sanitary articles as defined in claim 10.

The claims are an integral part of the teaching provided in relation to the invention.

### Brief Description of the Drawings

The present invention will now be described in detail with reference to the accompanying drawings, provided by way of non-limiting example, in which:
- Figure 1 is a schematic side view of an apparatus according to the present invention,
- Figures 2 and 3 are schematic top views illustrating an elastic film segment and an elastic thread segment, respectively, produced using a method and apparatus according to the present invention, and
- Figure 4 is a schematic top view along arrow IV in Figure 1, showing a chain of absorbent sanitary article blanks produced by a machine according to the present invention.

### Detailed Description

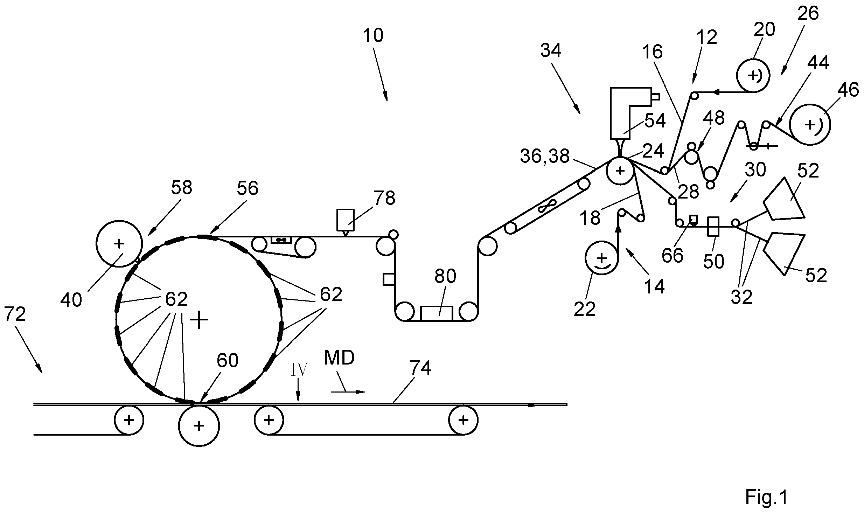

With reference to Figure 1, numeral 10 schematically indicates an apparatus for producing strip segments.

The apparatus 10 comprises a first and a second unwinding device 12, 14 configured to advance a first and a second non-woven fabric web 16, 18 unwound from respective reels 20, 22 toward an overlapping zone 24.

The apparatus 10 includes a film feeding unit 26 configured to feed a continuous elastic film 28 to the overlapping zone 24. The film feeding unit 26 includes an unwinding device 44 configured to unwind the continuous elastic film 28 from a reel 46 and a longitudinal stretching device 48 configured to stretch the continuous elastic film 28 elastically in a longitudinal direction. In the overlapping zone 24, the continuous elastic film 28, stretched elastically in the longitudinal direction, is fed between the first and second non-woven fabric webs 16, 18.

The apparatus 10 includes a thread feeding unit 30 configured to feed a plurality of elastic threads 32 to the overlapping zone 24. The thread feeding unit 30 includes a tension control device 50 configured to control the tensioning of the elastic threads 32 unwound from respective reels 52. In the overlapping zone 24, the elastic threads 32 are elastically stretched in the longitudinal direction and are arranged parallel to one another between the first and second non-woven fabric webs 16, 18.

The film feeding unit 26 and the thread feeding unit 30 are selectively operated to feed either the continuous elastic film 28 or the plurality of elastic threads 32 into the overlapping zone. When the film feeding unit 26 is operational, the thread feeding unit 30 is inactive, and vice versa.

The apparatus 10 includes a bonding unit 34 configured to bond together, in the overlapping zone 24, the first and second non-woven fabric webs 16, 18, overlapped on each other, and either the continuous elastic film 28 or the plurality of elastic threads 32, thereby selectively producing an elastic film strip 36 or an elastic thread strip 38. At the output of the bonding unit 34, the elastic film strip 36 or the elastic thread strip 38 is continuous.

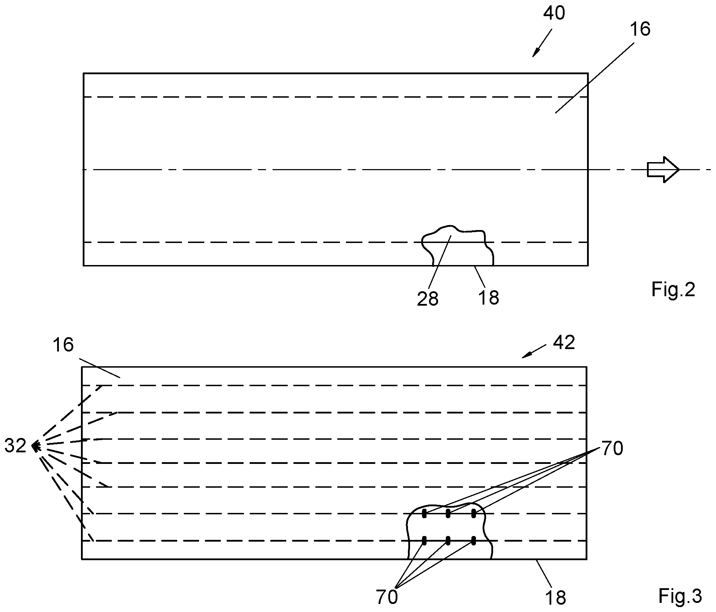

The bonding unit 34 may include an ultrasonic welding device 54 cooperating with an anvil roller 64 and configured to form a pattern of welding points 68 (Figure 2) that bond the continuous elastic film 28 to the first and second non-woven fabric webs 16, 18. The ultrasonic welding device 54 may also be configured to form anchoring welds 70 (Figure 3) that bond the elastic threads 32 to the first and second non-woven fabric webs 16, 18, as described, for example, in EP-A-3092997 by the same applicant. Alternatively, the apparatus 10 may include a glue dispenser 66 for intermittently applying glue to the elastic threads 32 to bond them to the first and second non-woven fabric webs 16, 18 using glue.

The apparatus 10 includes a cutting station 58 located downstream of the bonding unit 34, in which a cutting device 40 is configured to transversely cut the elastic film strip 36 or the elastic thread strip 38 to selectively form an array of elastic film segments 40 (Figure 2) or an array of elastic thread segments 42 (Figure 3).

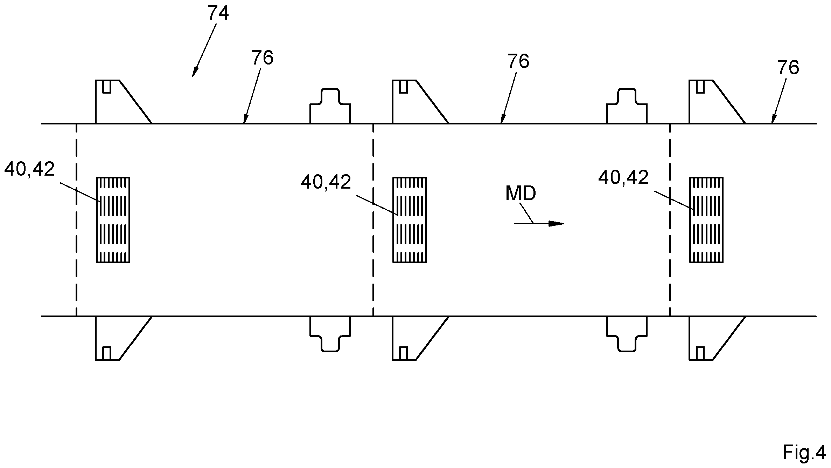

The apparatus 10 is integrated into a machine 72 for manufacturing absorbent sanitary articles, which produces a composite web 74 formed by a chain of absorbent sanitary article blanks 76 (Figure 4) advancing in a machine direction MD.

The apparatus 10 includes a transfer group 56 configured to transfer the elastic film segments 40 or elastic thread segments 42, formed downstream of the cutting device 40 at the cutting station 58, to an application station 60. In the application station 60, the elastic film segments 40 or elastic thread segments 42 are applied to the composite web 74 in positions spaced apart from each other in the machine direction MD.

The transfer group 56 may include a plurality of movable holders 62 configured to retain respective elastic film segments 40 or elastic thread segments 42, for example, by suction.

The cutting device 40 may be configured to transversely cut the end portions of the elastic film strip 36 or the elastic thread strip 38 while such end portions are retained by movable holders 62 of the transfer group 56.

The movable holders 62 may rotate 90° between the cutting station 58 and the application station 60 so that, in the application station 60, the elastic film segments 40 or elastic thread segments 42 are oriented orthogonally to the machine direction MD.

The apparatus 10 may include a glue dispenser 78 for applying glue to the elastic film strip 36 or the elastic thread strip 38 according to a predetermined pattern, so that when the elastic film segments 40 or elastic thread segments 42 are applied to the composite web 74 in the application station 60, they are bonded to the composite web 74 using glue.

The apparatus 10 may include an alignment device 80 positioned between the bonding unit 34 and the transfer group 56 to align the edges of the elastic film strip 36 or the elastic thread strip 38 with the holders of the transfer group 56.

In operation, the apparatus 10 implements a method for producing strip segments, comprising the steps of:
- advancing a first and a second non-woven fabric web 16, 18 unwound from respective reels 20, 22 toward an overlapping zone 24,
- selectively feeding either a longitudinally stretched continuous elastic film 28 or a plurality of longitudinally stretched elastic threads 32 to the overlapping zone 24 between the first and second non-woven fabric webs 16, 18,
- bonding, in the overlapping zone 24, the first and second non-woven fabric webs 16, 18, overlapped on each other, to either the continuous elastic film 28 or the plurality of elastic threads 32, thereby selectively producing an elastic film strip 36 or an elastic thread strip 38, and
- transversely cutting the elastic film strip 36 or the elastic thread strip 38 to selectively form an array of elastic film segments 40 or an array of elastic thread segments 42.

The continuous elastic film 28 may be unwound from a reel 46 and stretched elastically in the longitudinal direction.

The method may include controlling the tension of the elastic threads 32 unwound from respective reels 52.

The method may also include transferring the elastic film segments 40 or the elastic thread segments 42 from the cutting station 58 to the application station 60 and rotating the elastic film segments 40 or the elastic thread segments 42 during the transfer between the cutting station 58 and the application station 60.

From the foregoing description, it is clear that the apparatus and the method according to the present invention allow the production, within the same machine 72, of absorbent sanitary articles provided with either elastic film segments 40 or elastic thread segments 42, with minimal adjustments required to switch between the two types of production.

Naturally, without prejudice to the principles of the invention, construction details and embodiments may vary widely with respect to what has been described and illustrated without departing from the scope of the invention as defined by the claims that follow.

## Claims

1. An apparatus for producing strip segments, comprising:
- a first and a second unwinding device (12, 14) configured to advance a first and a second non-woven fabric web (16, 18) unwound from respective reels (20, 22) toward an overlapping zone (24),
- a film feeding unit (26) configured to feed a longitudinally stretched continuous elastic film (28) to said overlapping zone (24) between said first and second non-woven fabric webs (16, 18),
- a thread feeding unit (30) configured to feed a plurality of longitudinally stretched elastic threads (32) to said overlapping zone (24) between said first and second non-woven fabric webs (16, 18),
wherein the film feeding unit (26) and the thread feeding unit (30) are selectively operated to feed to said overlapping zone either said continuous elastic film (28) or said plurality of elastic threads (32),
- a bonding unit (34) configured to bond, in said overlapping zone (24), said first and second non-woven fabric webs (16, 18), overlapped on each other, to either said continuous elastic film (28) or said plurality of elastic threads (32), thereby selectively producing an elastic film strip (36) or an elastic thread strip (38), and
- a cutting station (58) located downstream of said bonding unit (34), comprising a cutting device (40) configured to transversely cut said elastic film strip (36) or said elastic thread strip (38) to selectively form an array of elastic film segments (40) or an array of elastic thread segments (42).

2. An apparatus according to claim 1, wherein said film feeding unit (26) comprises an unwinding device (44) configured to unwind said continuous elastic film (28) from a reel (46) and a longitudinal stretching device (48) configured to stretch said continuous elastic film (28) elastically in the longitudinal direction.

3. An apparatus according to claim 1, wherein said thread feeding unit (30) comprises a tension control device (50) configured to control the tensioning of elastic threads (32) unwound from respective reels (52).

4. An apparatus according to any of the preceding claims, wherein said bonding unit (34) comprises an ultrasonic welding device (54).

5. An apparatus according to any of the preceding claims, comprising a transfer group (56) configured to transfer said elastic film segments (40) or said elastic thread segments (42) from said cutting station (58) to an application station (60), wherein the transfer group (56) comprises a plurality of holders (62) configured to retain respective elastic film segments (40) or elastic thread segments (42), and wherein said holders (62) are rotatable by 90° between the cutting station (58) and the application station (60).

6. A method for producing strip segments, comprising the steps of:
- advancing a first and a second non-woven fabric web (16, 18) unwound from respective reels (20, 22) toward an overlapping zone (24),
- selectively feeding either a longitudinally stretched continuous elastic film (28) or a plurality of longitudinally stretched elastic threads (32) to said overlapping zone (24) between said first and second non-woven fabric webs (16, 18),
- bonding, in said overlapping zone (24), said first and second non-woven fabric webs (16, 18), overlapped on each other, to either said continuous elastic film (28) or said plurality of elastic threads (32), thereby selectively producing an elastic film strip (36) or an elastic thread strip (38), and
- transversely cutting said elastic film strip (36) or said elastic thread strip (38) to selectively form an array of elastic film segments (40) or an array of elastic thread segments (42).

7. A method according to claim 6, comprising unwinding said continuous elastic film (28) from a reel (46) and elastically stretching said continuous elastic film (28) in a longitudinal direction.

8. A method according to claim 6, comprising controlling the tensioning of elastic threads (32) unwound from respective reels (52).

9. A method according to any of the preceding claims, comprising transferring said elastic film segments (40) or said elastic thread segments (42) from a cutting station (58) to an application station (60) and rotating said elastic film segments (40) or said elastic thread segments (42) by 90° during the transfer between the cutting station (58) and the application station (60).

10. A machine for manufacturing absorbent sanitary articles comprising an apparatus for producing strip segments according to any of claims 1-5.
